# EUROPEAN PATENT APPLICATION

(11) **EP 3 967 364 A1**
(43) Date of publication of application: **16.03.2022**
(21) Application number: 21195674.3
(22) Date of filing: 09.09.2021
(51) Int. Cl.: A61N 1/36

(54) **MULTIDIMENSIONAL CODING OF STIMULATION PULSES**

(30) Priority: 14.09.2020 EP 20195989
(71) Applicant: Oticon Medical A/S, 2765 Smørum (DK)
(72) Inventor: WIJETILLAKE, Aswin Adris, 2765 Smørum (DK); DANG, Kai, Hubei Sheng, 441105 (CN); SEGOVIA MARTINEZ, Manuel, 06220 Vallauris (FR)
(74) Representative: Demant

(57) **Abstract**

An aspect of the disclosure is to provide a cochlear implant system comprising; a microphone unit configured to receive an acoustical input and provide an audio signal based on the acoustical input, and where the audio signal comprises multiple acoustic parameters, an electrode array including a plurality of electrodes configured to apply a plurality of stimulation pulses to auditory nerve fibers of a recipient of the cochlear implant system based on the audio signal, a memory unit including a plurality of multidimensional perceptual models where each multidimensional perceptual model includes perceptual information as a function of coding parameter groups, a perceptual modelling unit configured to retrieve at least one multidimensional perceptual model of the plurality of multidimensional perceptual models where the perceptual information of the at least one multidimensional perceptual model relates to a perceptual information of interest, an optimization unit configured to select a sequence of coding parameter groups by performing an optimization analysis of the retrieved multidimensional perceptual model and which fulfils at least one objective criterion while adhering to at least one constraint criterion; a signal processor unit including; a filter bank unit configured to process the audio signal into a plurality of band limited audio signals, a mapping unit configured to map each of coding parameter group of the sequence of coding parameter groups to each of the plurality of band limited audio signals based on an acoustic parameter of the multiple acoustic parameters, and a coding unit configured to code each of the plurality of band limited audio signals into a plurality of stimulation pulses based on the mapped coding parameter group and transfer the coded stimulation pulses to the plurality of electrodes.

## Description

### FIELD

The present disclosure relates to a cochlear implant system configured to code an audio signal based on multiple coding parameters.

### BACKGROUND

Known multichannel cochlear implant systems (CI) encode environmental sounds directly into a plurality of stimulation pulses using specific sound coding strategies, such as Continuous Interleaved Sampling (CIS) and Temporal Fine Structure (TFS). The coded plurality of stimulation pulses is then transmitted to the inner ear via electrical stimulations.

One of the targets of optimizing a cochlear implant system is to provide the recipient access to different perceptual aspects of hearing, e.g. pitch, timbre, loudness, perceived sound direction, and/or a distance to a sound source, which are controlled by coding parameters such as pulse rate, pulse width, pulse amplitude, pulse shape, pulse temporal onset pulse, perceived interaural timing difference, perceived interaural level differences, stimulation position, etc.

For example, perceptual information, such as loudness, pitch, timbre, sound direction and/or distance to a sound source can be coded into the plurality of stimulation pulses by changing one of the coding parameters. For example, for loudness the following coding parameters can be adjusted: pulse rate, pulse width, pulse amplitude, pulse shape.

In current implementations, the cochlear implant system chooses either the pulse amplitude or the pulse width as the variable parameter, while other coding parameters are kept constant. In this case, an electric dynamic range of an electrode of the electrode array and resolution of the perceived loudness may however be limited when the chosen coding parameter has certain restrictions on the patient. For example, when coding loudness through pulse amplitude with other parameters fixed, the usable range of amplitudes between the patients hearing threshold (T-level) and comfort level (C-level) may be narrow, and the CI system may be able to output a small number of pulse amplitude steps within that range.

In another example, changes in pitch may be coded by changing either the stimulation position (place coding) in the inner ear, the pulse stimulation rate or the pulse amplitude/width modulation rate. In Continuous Interleaved Sampling (CIS) based stimulation strategies, the stimulation pulses are delivered to the inner ear at a fixed rate. Therefore, the place coding method is the only considered path for delivering pitch information. Strategies that exploit the temporal fine structure (TFS) of the sound, may combine stimulation position with temporal coding by delivering stimulation pulses with different pulse stimulation rates to the inner ear according to the cochlear tonotopy. In this case, the place coding and the temporal coding may be highly correlated to the input signal frequency as in the normal cochlea: the frequency of the stimulation pulses monotonically increases from the most apical electrode to the most basal electrode of the electrode array in relation to the cochlear tonotopy, and there is no overlap between the frequency ranges assigned to each of the electrodes of the electrode array.

To summarize, all the examples describing how to code perceptual information into the stimulation pulses show that current strategies for the cochlear implant systems have only one degree of freedom, using either only one parameter or two highly correlated parameters for delivering a certain perceptual information to the patient via electrical stimulation. Therefore, the perceptual information is compressed to fit the electrical dynamic range of the chosen coding parameter, and the resolution and number of steps for that parameter within that dynamic range may be limited. This may lead to the degradation of perceptual outcomes and sound quality for the CI recipient, particularly when the coding parameter range on that certain recipient is further limited. Therefore, a cochlear implant system which can code perceptual information using all the available coding parameters in a joint way may improve the dynamic range and resolution of perceptual information while being robust to the limitations on any of the coding parameters.

### SUMMARY

An aspect of the disclosure is to provide a cochlear implant system that improves the patient's ability to perceive perceptual information coded into stimulation pulses provided by the cochlear implant system to the auditory nerve fibers of the patient.

The aspect of the disclosure is achieved by a cochlear implant system comprising a microphone unit configured to receive an acoustical input and provide an audio signal based on the acoustical input, and where the audio signal includes multiple acoustic parameters, and an electrode array including a plurality of electrodes configured to apply a plurality of stimulation pulses to auditory nerve fibers of a recipient of the cochlear implant system, and a memory unit including a plurality of multidimensional perceptual models where each multidimensional perceptual model includes perceptual information as a function of coding parameter groups.

A multidimensional perceptual model may for example include pitch (i.e. perceptual information) as a function of pulse rate (i.e. a first coding parameter) of the plurality of stimulation pulses and stimulation positions (i.e. a second coding parameter), i.e. electrode number of the plurality of electrodes of the electrode array. In this example, a pitch level is determined at a given pulse rate for a given electrode for the recipient of the system. The multidimensional perceptual pitch model may be generated during a fitting procedure where a number of an electrical stimulation patterns are presented to the recipient with both the electrode and pulse rate of those stimuli varied, and the recipient provides feedback about the perceived pitch of those stimuli. The fitting procedure may be performed at a hearing clinic, remotely from a hearing clinic or during use of the cochlear implant system in combination with an external input device that assists in the fitting procedure. The external input device, which may be a smartphone, leads the fitting procedure by introducing the recipient of the cochlear implant system to different inputs, and the recipient is able provided with feedback to the external input device.

The external input device may be provided by a computer, such as a fitting computer.

Furthermore the fitting procedure may be a computer-implemented system and/or a computer implemented method which comprises
an audio interface configured to provide an acoustical input to the microphone unit or an RF interface, such as Bluetooth, Low Energy Bluetooth, WIFI, telephone network or any other kind of communication links, of the cochlear implant system,
a recipient interface configured to receive recipient feedback based on the acoustical input,
a perceptual modelling logger configured to receive the recipient feedback from the recipient interface, acoustical parameters from the audio interface where the acoustical parameters characterizes the acoustical input.

Each of the plurality of multidimensional perceptual models may be customized for the recipient of the cochlear implant system via the fitting procedure, or, each of the plurality of multidimensional perceptual models may be predetermined based on similar multidimensional perceptual models of other recipients of a cochlear implant system. The customization of the plurality of multidimensional perceptual models may be done during the fitting procedure or during normal use of the cochlear implant system. The predetermined plurality of multidimensional perceptual models may be determined by interpolating between similar multidimensional perceptual models of the other recipients.

The customization and/or the predetermination of multidimensional perceptual models may be performed by a computer which may be connected to the cochlear implant system.

Predetermining the multidimensional perceptual models based on similar multidimensional perceptual models of other recipients results in a reduced time for generating multidimensional perceptual models in relation to the example where the model is customized.

Normal use of the cochlear implant system implies that the recipient is using the cochlear implant system for enhancing the recipient's ability to hear.

The cochlear implant system may comprise a perceptual modelling unit configured to retrieve at least one multidimensional perceptual model of the plurality of multidimensional perceptual models which relates to a perceptual information of interest. The perceptual information of interest may vary with respect to the acoustical input, i.e. variation in acoustical parameters of the acoustical input may result in variation in the perceptual information of interest. For example, for a given time period the perceptual information of interest is loudness but for a subsequent time period a fundamental frequency of the audio signal has changed such the perceptual information of interest will change from loudness to pitch.

Thereby, the perceptual modelling unit may be configured to retrieve at least one multidimensional perceptual model based on the multiple acoustic parameters.

The perceptual information of interest may be determined by a signal processing unit based on;
- a sound coding strategy used for coding the plurality of stimulation pulses,
- the acoustic environment information determined by an acoustic environment detector, and/or
- a sound processor program including setting parameters of the sound coding strategy.

The acoustical parameter may acoustically characterize the acoustical input including, frequencies and/or sound levels, envelope, energy at fundamental frequencies of the audio signal, spectral shape of energy of the acoustical input, detections of phonemic and/or phonetic, sound level, Crest factors, Signal-To-Noise ratio, and/or variation of amplitudes with respect to time.

The acoustical parameter may be measured or determined by a signal processor unit of the cochlear implant system.

The cochlear implant system may comprise an optimization unit configured to select a sequence of coding parameter groups by performing an optimization analysis of the retrieved at least one multidimensional perceptual model which fulfils at least one objective criterion while adhering to at least one constraint criterion.

The multidimensional perceptual model may comprise perceptual information as a function of coding parameter groups, where the sequence of coding parameter includes a selected part of the perceptual information as a function of coding parameter groups. The selected part fulfills the at least one objective criterion while adhering to at least one constraint criterion. The analysis may be performed during use of the cochlear implant system. For example, the at least one objective criterion is to obtain a sequence of electrode place and stimulation rate pairs that maximize the perceived pitch range for the recipient and the at least one constraint criterion could be that the pulse rate cannot exceed a defined maximum on any electrode. The optimization unit analyse the retrieved multidimensional perceptual model by going through numerous sequences of coding parameter groups within the model until the at least one objective criterion is obtained while adhering to the constraint criterion. More sophisticated optimization algorithms could be used to solve this optimization problem. The selected sequence of coding parameter groups which fulfills the at least one objective criterion will result in an optimal maximized perceived pitch range performance for the given moment the recipient listens to the received acoustical input.

The at least one objective criterion may be determined by the signal processor unit or by an external input device computer based on the perceptual information of interest.

The at least one objective criterion may be determined by the signal processor unit or by an external input device based on the perceptual information of interest, and wherein the at least one objective criterion is at least one of following;
- a maximized range and/or resolution of pitch of the recipient,
- a maximized loudness resolution of the recipient,
- a maximized timbre range and/or resolution perceived by the recipient
- a maximized range and/or resolution of perceivable sound source direction and /or
- a maximization of bimodal loudness fusion.

By including one of the mentioned objective criteria in determining the coding parameters will result in a coding of the stimulation pulses that improves the hearing capability of the recipient when using the cochlear implant system.

The at least one constraint criterion may be determined by the signal processor unit or by an external input device based on the perceptual information of interest, and where the at least one constraint criterion is at least one of following;
- a limitation of a coding parameter of the coding parameter group,
- a sound coding strategy for coding the plurality of stimulation pulses,
- a monotonically increasing pitch as a function of the sequence of coding parameter groups,
- a monotonically changing perceived angle of sound direction as a function of the sequence of coding parameter groups,
- a monotonically increasing loudness as a function of the sequence of coding parameter groups,
- a loudness that remains with the threshold of hearing and the maximum comfortable loudness, and/or
- a variation pattern of pitch or loudness from a most basal electrode of the electrode to a most apical electrode of the electrode array, and
- a maximum power output and consumption by the cochlear implant system
- a maximum allowed difference in values of a coding parameter between successive coding parameter groups in a sequence of coding parameter groups
- a maximum allowed change in perceptual information of interested elicited by successive coding parameter groups in a sequence of coding parameter groups.

The constrains are not only preventing uncomfortable sensations for the patient, but also to guide the optimization algorithm to a sensible solution, by narrowing down the pool of solutions. Without constraint criteria, the optimization algorithm could reach a huge number of solutions, many of which are not viable. For example, without any constraint criteria for the pitch case, the algorithm could output a sequence of stimulation parameters that causes non-monotonic changes in pitch.

By including one of the mentioned constraint criteria in determining the coding parameters will result in a coding of the stimulation pulses that improves the hearing capability of the recipient without being uncomfortable when using the cochlear implant system. For example, the loudness can be increased to a level where the recipient is able to hear more but is not comfortable, i.e. the loudness is above the C-level, and by applying a constraint criterion which defines that the loudness should not be increased above the C-level the improvement of the hearing capability is done without being uncomfortable for the recipient.

If the at least one objective criterion is to maximize range of pitch, the optimization unit is configured to perform the optimization analysis by selecting a sequence of coding parameter groups that fulfills the at least one objective criterion and the at least one constraint criterion. The coding parameters may include pulse stimulation rate and stimulating electrode. The constraint criteria for the optimization method could, for example, include i) the perceived pitch elicited by the selected sequence of parameter groups must increase monotonically and ii) the electrode index of each group in the sequence must be greater than or equal to the electrode index of the preceding groups in the sequence. The selected sequence of coding parameter groups is then coded into the plurality of stimulation pulses.

The cochlear implant system may comprise the signal processor unit which includes a filter bank unit configured to process the audio signal into a plurality of band limited audio signals, and a mapping unit configured to map each of coding parameter group of the sequence of coding parameter groups to each of the plurality of band limited audio signals based on an acoustic parameter of the multiple acoustic parameters. Each of the band limited audio signal includes one or more frequencies of the audio signal within a frequency range defined by a bandwidth of a bandpass filter of the filter bank.

The signal processor unit may comprise a coding unit configured to code each of the plurality of band limited audio signals into a plurality of stimulation pulses based on the mapped coding parameter group and transfer the coded stimulation pulses to the plurality of electrodes.

Each electrode of the electrode array may be allocated to a frequency range predetermined via a fitting procedure or determined dynamically by the signal processor unit based on the audio input, and each of the stimulation pulses is allocated to each electrode of the electrode array when the frequency of the stimulation pulse is within the frequency range of the electrode.

The at least one objective criterion may be to maximize the range of loudness and the at least one constraint criterion may be to maximize the range of loudness for a specific number of electrodes of the plurality of electrodes within an electrical dynamic range defined between the hearing threshold (T-level) and the hearing comfortable level (C-level) of the recipient. The optimization unit analyses numerous of sequence of coding parameters using optimization techniques to select a sequence of coding parameters which fulfills the objective criterion while adhering to the constraint criterion, and the coding unit may code each of the plurality of band limited audio signals based on the coding parameter group of the selected sequence of coding parameter groups. And in this specific example, the recipient will experience an improved hearing capability.

The hearing threshold (T level) defines the lowest level of electrical stimulation current necessary for a person to perceive a sound, and the hearing comfortable level (C-level) defines the highest electoral level that will be output by the implant, where that level elicits a loudness that comfortable.

C level may be defined and set different. Sometimes it is defined as the maximum electrical level that will still produce a comfortable sensation (i.e. it is just below the level that would cause discomfort). This level is sometimes also called the 'maximum comfort' (MC) level. Other times that patient is simply asked to indicate a level that is loud and comfortable, which may lower than the MC.
The at least one constraint criterion may be a limitation of a coding parameter of the coding stimulation group determined by a min and max value of the coding parameter.

The optimization analysis for selecting the sequence of coding parameter groups may comprise selecting a first sequence of coding parameter groups and selecting a second sequence of coding parameter groups, and then, selecting either the first or the second sequence of coding parameter groups based on which of the first or the second sequence of coding parameter groups fulfills the at least one objective criterion while adhering to the at least one constraint criterion.

The optimization analysis may be performed in an iterative manner to obtain an optimal sequence of coding parameter groups of the first and second sequence of coding parameter groups.

The perceptual information of interest may vary with respect to the acoustical input, and thereby, different perceptual models will be retrieved along with the variation in the acoustical input. Thereby, for minimizing the computational power and delay for selecting the models, it is an advantage that the perceptual modelling unit may be configured to retrieve a group of multidimensional perceptual models of the plurality of multidimensional perceptual models which relates to one or more perceptual information of interests.

The optimization unit may be configured to shift between the retrieved multidimensional perceptual models of the group of multidimensional perceptual models based on a criteria.

The criteria may be one of following;
- a sound coding strategy used for coding the plurality of stimulation pulses,
- the acoustic environment information determined by the acoustic environment detector according to claim 6, and/or
- a sound processor program including setting parameters of the sound coding strategy.

A coding parameter of a coding parameter group may be a selection of at least one or more of following;
- a pulse rate of the plurality of stimulation pulses provided to an electrode of the electrode array,
- a pulse width of each of the plurality of stimulation pulses provided to an electrode of the electrode array,
- a pulse amplitude of each of the plurality of stimulation pulses to be provided to an electrode of the electrode array,
- a pulse shape of a stimulation pulse of the plurality of stimulation pulses to be provided to an electrode of the electrode array,
- a limited number of electrodes of the electrode array to be activated by a stimulation pulse,
- a temporal onset used for sampling the audio signal to be coded and provided to an electrode of the electrode array,
- an interaural timing difference between either onset timings of or the envelope encoded by a group of individual stimulation pulses of another plurality of stimulation pulses received from another cochlear implant system arranged at an opposite ear to an ear of which the cochlear implant system is arranged at,
- an interaural timing difference between a group of individual stimulation pulses of the plurality of stimulation pulses and either the event timings in the acoustic signal from a hearing aid or event timings in the vibro-acoustic signal of a bone conduction device arranged at an opposite ear to an ear of which the cochlear implant system is arranged at,
- an interaural level difference between a group of individual stimulation pulses of the plurality of stimulation pulses and another group of individual stimulation pulses of another plurality of stimulation pulses received from another cochlear implant system arranged at an opposite ear to an ear of which the cochlear implant system is arranged at,
- an interaural level/loudness difference between a group of individual stimulation pulses of the plurality of stimulation pulses and either an acoustic signal received from a hearing aid or a vibro-acoustic signal received from bone conduction device arranged at an opposite ear to an ear of which the cochlear implant system is arranged at,
a stimulation position provided by selection of one or more electrodes of the electrode array to receive the plurality of stimulation pulses.

The cochlear implant system may comprise an external input device, an external part, and an implantable unit; and wherein the memory unit, the perceptual modelling unit and the optimization unit may be part of the external input device, and the microphone unit and the signal processor may be part of the external part, and the electrode array may be part of the implantable unit, where the external part may be configured to communicate with the implantable unit via a transcutaneously link, and the external input device may be configured to communicate with the external part and/or the implantable unit; or wherein the memory unit, the perceptual modelling unit, the optimization unit, the microphone unit and the signal processor may be part of the external part and the electrode array may be part of the implantable unit, where the external part may be configured to communicate with the implantable unit.

### BRIEF DESCRIPTION OF DRAWINGS

The aspects of the disclosure may be best understood from the following detailed description taken in conjunction with the accompanying figures. The figures are schematic and simplified for clarity, and they just show details to improve the understanding of the claims, while other details are left out. Throughout, the same reference numerals are used for identical or corresponding parts. The individual features of each aspect may each be combined with any or all features of the other aspects. These and other aspects, features and/or technical effect will be apparent from and elucidated with reference to the illustrations described hereinafter in which:
- Fig. 1: illustrates a cochlear implant system;
- Figs. 2A to 2D: illustrate a cochlear implant system;
- Figs. 3A to 3C: illustrate a multidimensional perceptual model;
- Figs 4A and 4B: illustrate another example of a multidimensional perceptual model; and
- Figs. 5: illustrates another example of a multidimensional perceptual model.

### DETAILED DESCRIPTION

The detailed description set forth below in connection with the appended drawings is intended as a description of various configurations. The detailed description includes specific details for the purpose of providing a thorough understanding of various concepts. However, it will be apparent to those skilled in the art that these concepts may be practiced without these specific details. Several aspects of the apparatus and methods are described by various blocks, functional units, modules, components, etc. (collectively referred to as "elements"). Depending upon particular application, design constraint criteria or other reasons, these elements may be implemented using other equivalent elements.

The hearing aid that is adapted to improve or augment the hearing capability of a recipient by receiving an acoustic signal from a recipient's surroundings, generating a corresponding audio signal, possibly modifying the audio signal and providing the possibly modified audio signal as an audible signal to at least one of the recipient's ears. Such audible signals may also be provided in the form of an acoustic signal transferred as mechanical vibrations to the recipient's inner ears through bone structure of the recipient's head.

The hearing aid is adapted to be worn in any known way. This may include arranging a unit of the hearing aid attached to a fixture implanted into the skull bone such as in a Bone Anchored Hearing Aid or at least a part of the hearing aid may be an implanted part.

A "hearing system" or a "cochlear implant system" refers to a system comprising one or two hearing aids, one or two cochlear implants, and a "binaural hearing system" refers to a system comprising two hearing aids or two cochlear implants where the devices are adapted to cooperatively provide audible signals to both of the recipient's ears or the hearing aid of bone conduction type or an acoustical hearing aid may be part of a bimodal system comprising a cochlear implant and a hearing aid or a bone conduction hearing aid. The system may further include an external device(s) that communicates with at least one hearing aid, the external device affecting the operation of the hearing aids and/or benefitting from the functioning of the hearing aids. A wired or wireless communication link between the at least one hearing aid and the external device is established that allows for exchanging information (e.g. control and status signals, possibly audio signals) between the at least one hearing aid and the external device. Such external devices may include at least one of remote controls, remote microphones, audio gateway devices, mobile phones, public-address systems, car audio systems or music players or a combination thereof. The audio gateway is adapted to receive a multitude of audio signals such as from an entertainment device like a TV or a music player, a telephone apparatus like a mobile telephone or a computer, a PC. The audio gateway is further adapted to select and/or combine an appropriate one of the received audio signals (or combination of signals) for transmission to the at least one hearing aid. The remote control is adapted to control functionality and operation of the at least one hearing aids. The function of the remote control may be implemented in a SmartPhone or other electronic device, the SmartPhone/ electronic device possibly running an application that controls functionality of the at least one hearing aid.

In general, a hearing aid or a cochlear implant includes i) an input unit such as a microphone for receiving an acoustic signal from a recipient's surroundings and providing a corresponding input audio signal, and/or ii) a receiving unit for electronically receiving an input audio signal. The hearing aid further includes a signal processing unit for processing the input audio signal and an output unit for providing an audible signal to the recipient in dependence on the processed audio signal.

The input unit may include multiple input microphones, e.g. for providing direction-dependent audio signal processing. Such directional microphone system is adapted to enhance a target acoustic source among a multitude of acoustic sources in the recipient's environment. In one aspect, the directional system is adapted to detect (such as adaptively detect) from which direction a particular part of the microphone signal originates. This may be achieved by using conventionally known methods. The signal processing unit may include amplifier that is adapted to apply a frequency dependent gain to the input audio signal. The signal processing unit may further be adapted to provide other relevant functionality such as compression, noise reduction, etc. The output unit may include an output transducer for providing mechanical vibrations either transcutaneously or percutaneously to the skull bone.

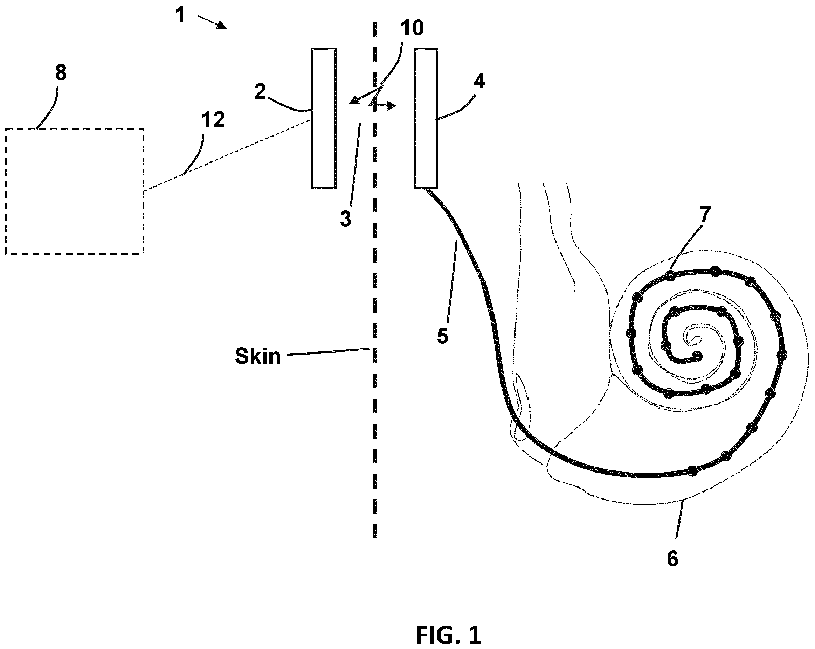

Fig. 1 illustrates a cochlear implant system which includes an external part (2) and an implantable unit 3 which is connected to an electrode array 5 including a plurality of electrodes 7, where each of the electrodes is configured to provide an electrical stimulation to auditory nerve fibers of the cochlear for enhancing the recipient's hearing capability. In the specific illustrations, the electrode array 5 is inserted into the cochlea 6 of a recipient of the system. The external part is configured to communicate transcutaneously 10 to the implantable unit. Optionally, the external part may be configured to communicate with an external input device 8, such as a smartphone or a fitting computer, via a radio frequency communication link 12 based on a short or long range communication protocol, such as Bluetooth , Bluetooth Low Energy, Wireless Fidelity (WIFI), telephone network (3G, 4G, 5G, 6G etc.).

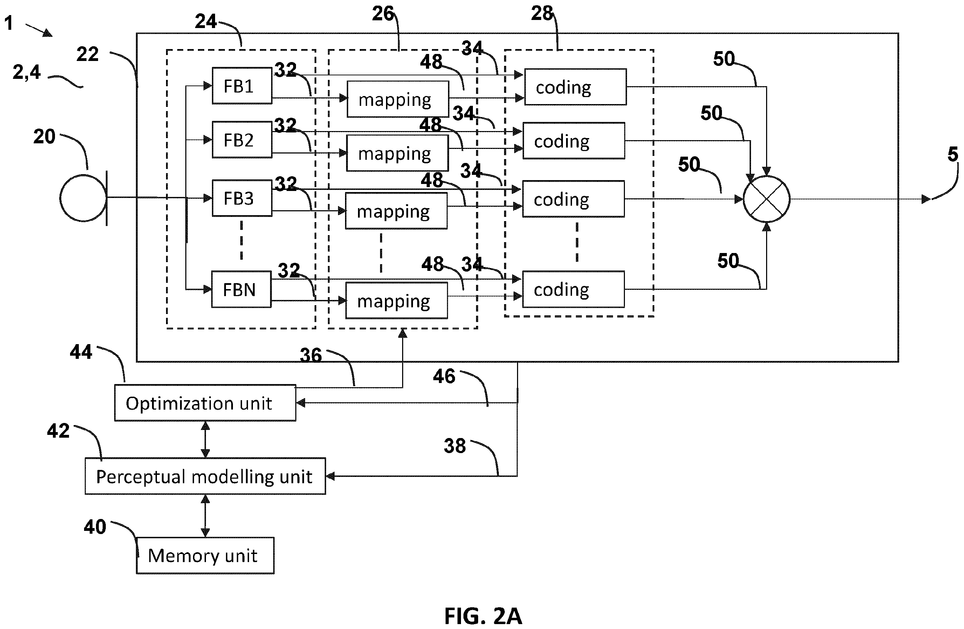

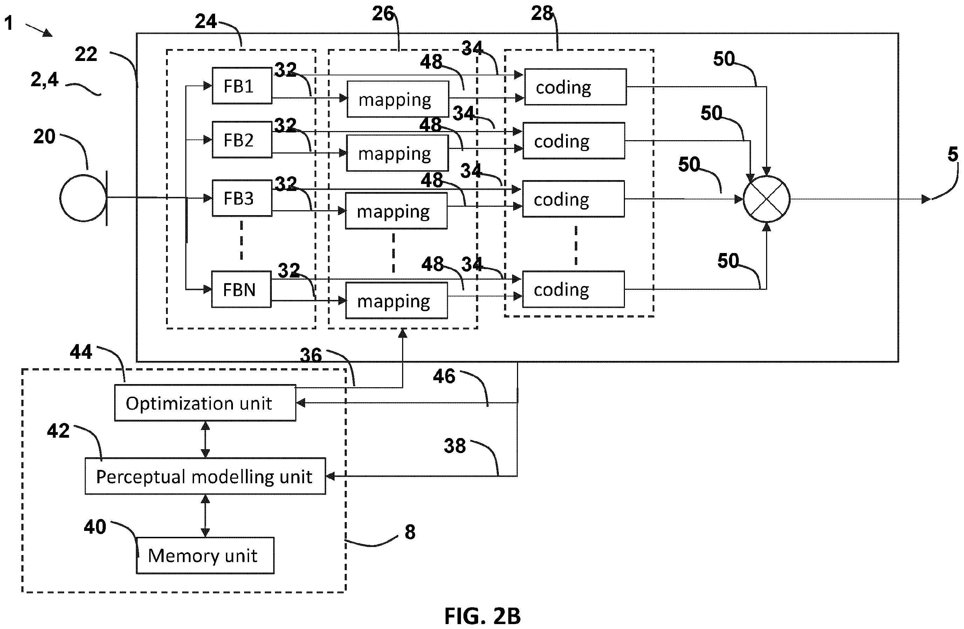

FIGS 2A to 2D illustrate an example of the cochlear implant system 1 where the external part 2 or the implantable unit 4 includes a microphone unit configured to receive an acoustical input and provide an audio signal based on the acoustical input, and where the audio signal includes multiple acoustic parameters. The system further includes an electrode array 5 which includes a plurality of electrodes configured to apply a plurality of stimulation pulses to auditory nerve fibers of a recipient of the cochlear implant system based on the audio signal. The system 1 includes a signal processor 22 which includes a filter bank 24 configured to process the audio signal into a plurality of band limited audio signals, and in the specific example, the filter bank 24 includes multiple bandpass filters (FB1, FB2, FB3 and FBN) where each of the bandpass filters is configured to pass through a band limited audio signal 35 defined as a part of the audio signal having a frequency range defined by the bandwidth of the bandpass filter. Furthermore, the signal processor 22 is configured to determine an acoustic parameter 32 for each of the band limited audio signals 34, and in the specific example, the acoustic parameter 32 is the frequency range of the band limited audio signal 34. In FIG. 2A, the external part 2 comprises a perceptual modelling unit 42 which is configured to retrieve at least one multidimensional perceptual model 41 of a plurality of multidimensional perceptual models stored in a memory unit 40, and where the perceptual information of the retrieved model relates to a perceptual information of interest 38, which in this example, is determined by the signal processor 22. The perceptual modelling unit 42 may be configured to retrieve a group of multidimensional perceptual models of the plurality of multidimensional perceptual models which relates to one or more perceptual information of interests. The external part 2 includes further an optimization unit 44 configured to select a sequence of coding parameter groups by performing an optimization analysis of the retrieved multidimensional perceptual model and which fulfils at least one objective criterion while adhering to at least one constraint criterion. In this example, the at least one objective criterion 46 and the at least one constraint criterion 46 are received from the signal processor unit 22. The optimization unit may be configured to shift between the retrieved multidimensional perceptual models of the group of multidimensional perceptual models based on a criteria, for example s sound coding strategy used for coding the plurality of stimulation pulses. The sequence of coding parameter groups is the transferred 36 to a mapping unit 26 of the signal processor unit 22, where the mapping unit 26 is configured to map each of coding parameter group 48 of the sequence of coding parameter groups to each of the plurality of band limited audio signals 34 based on an acoustic parameter of the multiple acoustic parameters

The signal processor unit 22 includes a mapping unit 26 configured to map each of coding parameter group 48 of the sequence of coding parameter groups 36 to each of the plurality of band limited audio signals 34 based on an acoustic parameter 32 of the multiple acoustic parameters.

The signal processor unit 22 includes a coding unit configured to code each of the plurality of band limited audio signals 34 into a plurality of stimulation pulses based on the mapped coding parameter group 48 and transfer the coded stimulation pulses 50 to the electrode array 5.

In another example the perceptual modelling unit and the optimization unit may be combined into one single unit.

FIG: 2B illustrates an example where the memory unit 40, the perceptual modelling unit 42 and the optimization unit 44 are arranged in an external input device 8.

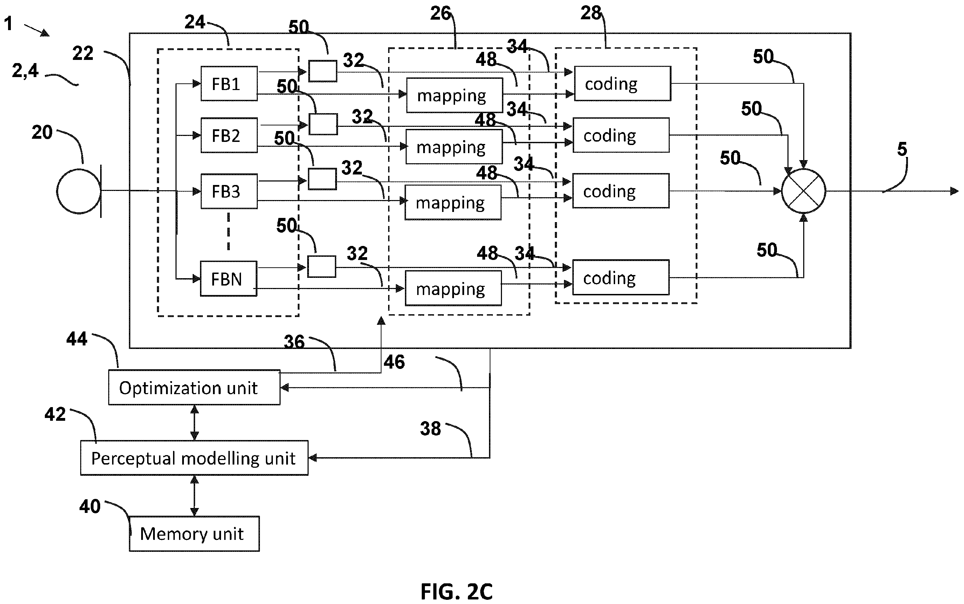

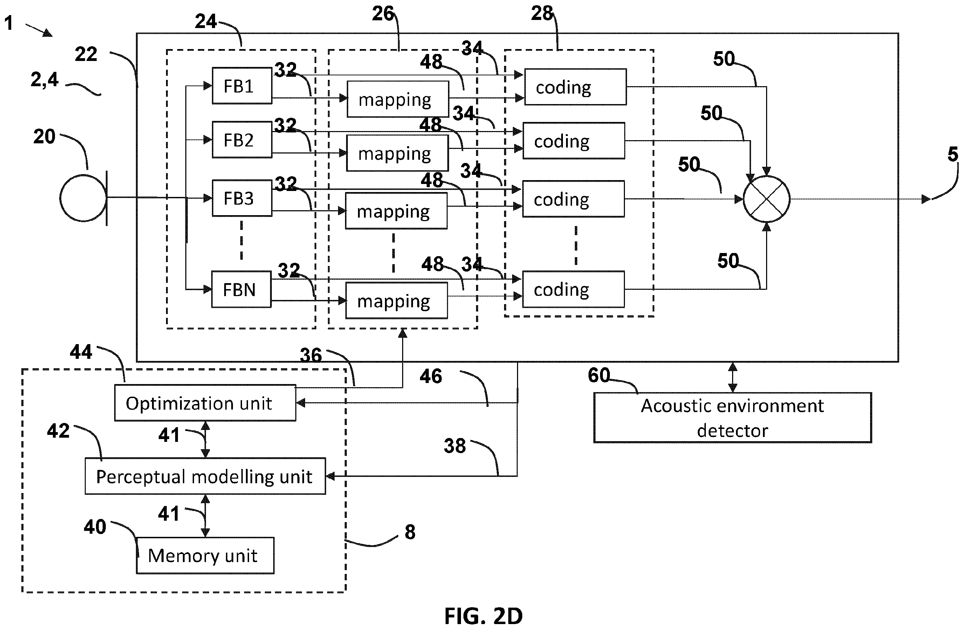

FIGS: 2C and 2D illustrate an example where the system 1 includes an acoustic environment detector 60 which is connected to the signal processor unit 22 and configured to receive the audio signal, and the acoustic environment detector 60 is configured to determine an acoustic environment information 52 of the audio signal, and wherein the signal processor unit 22 is configured to determine the at least one constraint criterion based on the acoustic environment information 52. In FIG. 2C, the acoustic environment detector 60 is connected to each of the bandpass filter (FB1 to FBN) of the filter bank 24 for detecting the acoustic environment information 52 for each of the band limited audio signal 34, and the signal processor unit 22 is then configured to apply multiple constraint criteria to the optimization unit, where each of the constraint criteria is relevant for a part of the retrieved multidimensional perceptual model. For example, each constraint criterion is relevant for a part of the retrieved multidimensional model which relates to frequencies which relates to the frequency range of the band limited audio signal 34 which the constraint criterion is determined on. In FIG. 2D, the acoustic environment detector 60 is connected to the signal processor such that the at least one constraint criterion is determined by the audio signal, i.e. a full frequency range of the audio signal.

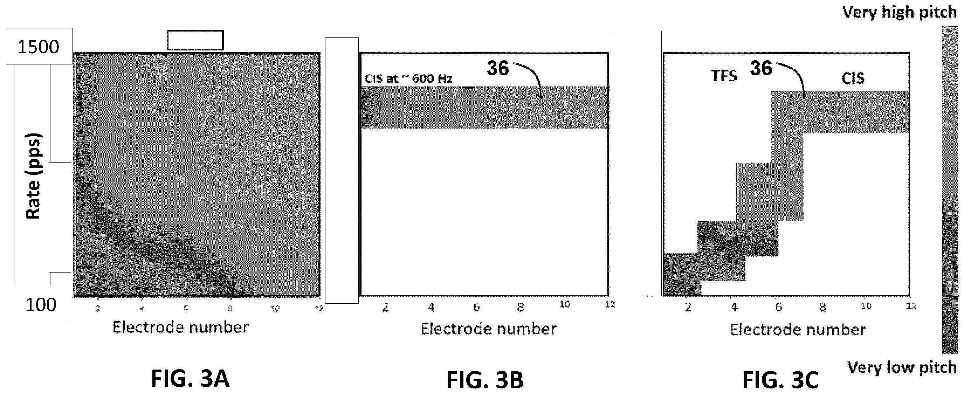

FIGS. 3A to 3C illustrate a multidimensional perceptual model including perceptual information as a function of coding parameter groups. In the specific example, the perceptual information is pitch and each coding parameter group includes a combination of pulse rate and electrode number. Furthermore, the at least one objective criterion is to maximize range of pitch of the recipient. FIG. 3A illustrates the multidimensional perceptual model, where the level of pitch is indicated by the color scalar on the figure. FIG: 3B illustrate an example of a selected sequence of coding parameter groups where the at least one constraint criterion contains three constraint criteria;
1. Rate must be constant;
2. The pitch must increase monotonically from the first set of parameters in the sequence to the last; and
3. Electrode cannot change by more than 1 electrode place from one element of the sequence to the next.
for a sound coding strategy Continuous-Interleaved Sampling (CIS). FIG: 3C illustrates an example of a selected sequence of coding parameter groups where five constraint criteria are applied;
1. Rate must be constant when the electrode index is greater than 7;
2. The pitch must increase monotonically from the first set of parameters in the sequence to the last;
3. Pitch cannot increase by more than x from one element of the sequence to the next;
4. Rate cannot change by more than y from one element of the sequence to the next; and
5. Electrode cannot change by more than 1 electrode place from one element of the sequence to the next.

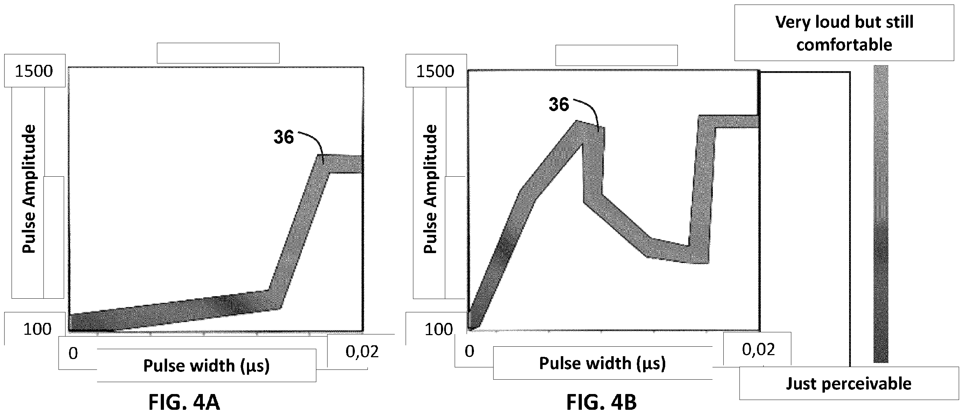

FIGS 4A and 4B illustrate another example of the multidimensional perceptual model. In the specific example, the perceptual information is loudness and each coding parameter group includes a combination of pulse amplitude and pulse width. In FIG. 4A the at least one objective criterion is to maximize the length, i.e. maximize the range, of both coding parameters sets in the sequence. The constraint criteria are as following;
1. Perceived pitch must increase monotonically across the whole sequence of stimulation parameters;
2. Stimulation cannot occur on electrode number 6;
3. Perceived pitch cannot increase by more than x from one set in the sequence to the next;
4. Pulse rate cannot change by more than y between successive coding parameter groups in the sequence, when electrode index is less than 6;
5. Pulse rate cannot change by more than y between successive coding parameter groups in the sequence, when electrode index is greater than 6;
6. Electrode index cannot change by more than 1 between successive coding parameter groups in the sequence, when electrode index is less than 6; and
7. Electrode index cannot change by more than 1 between successive coding parameter groups in the sequence, when electrode index is greater than 6;

In FIG: 4B, the at least one objective criterion is to maximize the length, i.e. maximize the range, of both coding parameters sets in the sequence. The constraint criteria are as following;
1. Loudness should monotonically increase as a function of the sequence;
2. Loudness cannot increase by more than x from one parameter set to the next in the sequence;
3. The pulse amplitude cannot change by more than y from one parameter set to the next in the sequence; and
4. The pulse width cannot change by more than z from one parameter set to the next in the sequence.

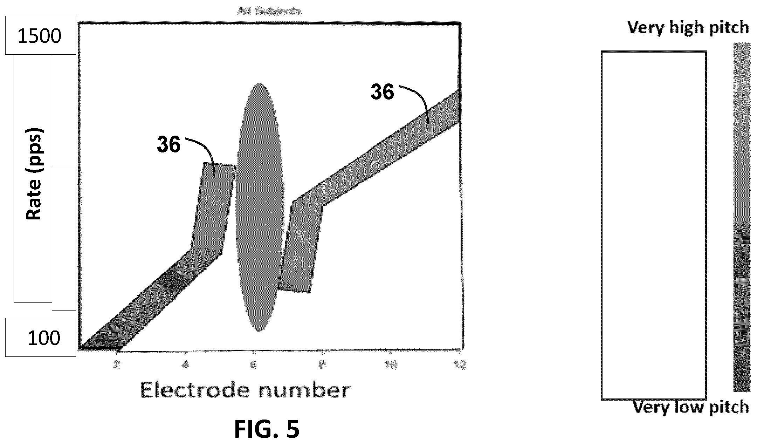

FIG. 5 illustrates an example where an electrode is deactivated (marked with elliptical marker), and in this case, the pulse rate on other electrodes of the electrode array are coded to cover the same pitch range as if the electrode was not deactivated. In the example, the at least one objective criterion is to maximize the pitch range, and the at least one constraint criterion is the deactivation of one or more electrode. The coding parameter group of the selected sequence includes pulse rate and electrode number.

As used, the singular forms "a," "an," and "the" are intended to include the plural forms as well (i.e. to have the meaning "at least one"), unless expressly stated otherwise. It will be further understood that the terms "includes," "comprises," "including," and/or "comprising," when used in this specification, specify the presence of stated features, elements, components, and/or steps but do not preclude the presence or addition of one or more other features, elements, components, and/or steps thereof. It will also be understood that when an element is referred to as being "connected" or "coupled" to another element, it can be directly connected or coupled to the other element, but an intervening element may also be present, unless expressly stated otherwise. As used herein, the term "and/or" includes any and all combinations of one or more of the associated listed items. The steps of any disclosed method are not limited to the exact order stated herein, unless expressly stated otherwise.

It should be appreciated that reference throughout this specification to "one embodiment" or "an embodiment" or "an aspect" or features included as "may" means that a particular feature, structure or characteristic described in connection with the embodiment is included in at least one embodiment of the disclosure. Furthermore, the particular features, structures or characteristics may be combined as suitable in one or more embodiments of the disclosure. The previous description is provided to enable any person skilled in the art to practice the various aspects described herein. Various modifications to these aspects will be readily apparent to those skilled in the art, and the generic principles defined herein may be applied to other aspects.

The scope should be judged in terms of the claims that follow.

## Claims

1. A cochlear implant system comprising:
- a microphone unit configured to receive an acoustical input and provide an audio signal based on the acoustical input, and where the audio signal comprises multiple acoustic parameters,
- an electrode array including a plurality of electrodes configured to apply a plurality of stimulation pulses to auditory nerve fibers of a recipient of the cochlear implant system based on the audio signal,
- a memory unit including a plurality of multidimensional perceptual models where each multidimensional perceptual model includes perceptual information as a function of coding parameter groups,
- a perceptual modelling unit configured to retrieve at least one multidimensional perceptual model of the plurality of multidimensional perceptual models where the perceptual information of the at least one multidimensional perceptual model relates to a perceptual information of interest,
- an optimization unit configured to select a sequence of coding parameter groups of the retrieved multidimensional perceptual model, and where the selected sequence of coding parameter groups fulfils at least one objective criterion while adhering to at least one constraint criterion,
- a signal processor unit including;
∘ a filter bank unit configured to process the audio signal into a plurality of band limited audio signals,
∘ a mapping unit configured to map each of coding parameter group of the sequence of coding parameter groups to each of the plurality of band limited audio signals based on an acoustic parameter of the multiple acoustic parameters, and
∘ a coding unit configured to code each of the plurality of band limited audio signals into a plurality of stimulation pulses based on the mapped coding parameter group and transfer the coded stimulation pulses to the plurality of electrodes.

2. A cochlear implant system according to claim 1, where the perceptual information is at least one of following; pitch, timbre, loudness, perceived sound direction, and/or a distance to a sound source.

3. A cochlear implant system according to any of the previous claims, wherein each of the plurality of multidimensional perceptual models is customized for the recipient of the cochlear implant system via a fitting procedure, or, each of the plurality of multidimensional perceptual models is predetermined based on similar multidimensional perceptual models of other recipients of a cochlear implant system.

4. A cochlear implant system according to any of the previous claims, wherein a coding parameter of a coding parameter group is a selection of at least one or more of following;
• a pulse rate of the plurality of stimulation pulses provided to an electrode of the electrode array,
• a pulse width of each of the plurality of stimulation pulses provided to an electrode of the electrode array,
• a pulse amplitude of each of the plurality of stimulation pulses to be provided to an electrode of the electrode array,
• a pulse shape of a stimulation pulse of the plurality of stimulation pulses to be provided to an electrode of the electrode array,
• a limited number of electrodes of the electrode array to be activated by a stimulation pulse,
• a temporal onset used for sampling the audio signal to be coded and provided to an electrode of the electrode array,
• an interaural timing difference between either onset timings of or the envelope encoded by a group of individual stimulation pulses of another plurality of stimulation pulses received from another cochlear implant system arranged at an opposite ear to an ear of which the cochlear implant system is arranged at,
• an interaural timing difference between a group of individual stimulation pulses of the plurality of stimulation pulses and either the event timings in the acoustic signal from a hearing aid or event timings in the vibro-acoustic signal of a bone conduction device arranged at an opposite ear to an ear of which the cochlear implant system is arranged at,
• an interaural level difference between a group of individual stimulation pulses of the plurality of stimulation pulses and another group of individual stimulation pulses of another plurality of stimulation pulses received from another cochlear implant system arranged at an opposite ear to an ear of which the cochlear implant system is arranged at,an interaural level/loudness difference between a group of individual stimulation pulses of the plurality of stimulation pulses and either an acoustic signal received from a hearing aid or a vibro-acoustic signal received from bone conduction device arranged at an opposite ear to an ear of which the cochlear implant system is arranged at,
• a stimulation position provided by selection of one or more electrodes of the electrode array to receive the plurality of stimulation pulses.

5. A cochlear implant system according to any of the previous claims, where the at least one objective criterion is determined by the signal processor unit based on the perceptual information of interest, and wherein the at least one objective criterion is at least one of following;
• a maximized range and/or resolution of pitch of the recipient,
• a maximized loudness resolution of the recipient,
• a maximized timbre range and/or resolution perceived by the recipient,
• a maximized range and/or resolution of perceivable sound source direction and /or
• a maximization of bimodal loudness fusion.

6. A cochlear implant system according to any of the previous claims, wherein the cochlear implant system includes an acoustic environment detector which is connected to the signal processor unit and configured to receive the audio signal, and the acoustic environment detector is configured to determine an acoustic environment information of the audio signal, and wherein the signal processor unit is configured to determine the at least one constraint criterion based on the acoustic environment information.

7. A cochlear implant system according to claim 1 to 5 and/or 6, where the at least one constraint criterion is controlled by the signal processor unit, the optimization unit based on the perceptual information of interest, and where the at least one constraint criterion is at least one of following;
• a limitation of a coding parameter of the coding parameter group,
• a sound coding strategy for coding the plurality of stimulation pulses,
• a monotonically increasing pitch as a function of the sequence of coding parameter groups,
• a monotonically changing perceived angle of sound direction as a function of the sequence of coding parameter groups,
• a monotonically increasing loudness as a function of the sequence of coding parameter groups
• a loudness that remains with the threshold of hearing and the maximum comfortable loudness, and/or
• a variation pattern of pitch or loudness from a most basal electrode of the electrode to a most apical electrode of the electrode array, and
• a maximum power output and consumption by the cochlear implant system
• a maximum allowed difference in values of a coding parameter between successive coding parameter groups in a sequence of coding parameter groups
• a maximum allowed change in perceptual information of interested elicited by successive coding parameter groups in a sequence of coding parameter groups.

8. A cochlear implant system according to an of claims 1 to 5 or claim 6, wherein the perceptual information of interest is determined by the signal processing unit based on;
• a sound coding strategy used for coding the plurality of stimulation pulses,
• the acoustic environment information determined by the acoustic environment detector, and/or
• a sound processor program including setting parameters of the sound coding strategy.

9. A cochlear implant system according to any of the previous claims, where the optimization analysis for selecting the sequence of coding parameter groups comprising;
• selecting a first sequence of coding parameter groups,
• selecting a second sequence of coding parameter groups,
• selecting either the first or the second sequence of coding parameter groups based on which of the first or the second sequence of coding parameter groups fulfills the at least one obj ective criterion and the at least one constraint criterion.

10. A cochlear implant system according to claim 9, wherein the optimization analysis is performed in an iterative manner to obtain an optimal sequence of coding parameter groups of the first and second sequence of coding parameter groups.

11. A cochlear implant system according to any of the previous claims, where the perceptual modelling unit is configured to retrieve a group of multidimensional perceptual models of the plurality of multidimensional perceptual models which relates to one or more coding parameters to one or more perceptual information of interests, and the optimization unit is configured to shifts between the retrieved multidimensional perceptual models of the group of multidimensional perceptual models based on a criteria.

12. A cochlear implant system according to claim 11, wherein the criteria is one of following;
• a sound coding strategy used for coding the plurality of stimulation pulses,
• the acoustic environment information determined by the acoustic environment detector according to claim 6, and/or
• a sound processor program including setting parameters of the sound coding strategy.

13. A cochlear implant system according to any of the previous claims, comprising an external input device, an external part, and an implantable unit; and wherein cochlear the memory unit, the perceptual modelling unit and the optimization unit are part of the external input device, and the microphone unit and the signal processor are part of the external part, and the electrode array is part of the implantable unit, where the external part is configured to communicate with the implantable unit via a transcutaneously link, and the external input device is configured to communicate with the external part and/or the implantable unit; or wherein the memory unit, the perceptual modelling unit, the optimization unit, the microphone unit and the signal processor are part of the external part and the electrode array is part of the implantable unit, where the external part is configured to communicate with the implantable unit.

14. A cochlear implant system according to claim 13, wherein the external part is a behind the ear hearing aid and/or an external part which is configured to be arranged on the head by a magnetic force.

15. A cochlear implant system according to any of claims 13 to 14, wherein the external input device is a smartphone or a computer.
